(19) Europäisches Patentamt
European Patent Office
Office européen des brevets

(11) **EP 1 113 726 B1**

(12) **EUROPEAN PATENT SPECIFICATION**

(45) Date of publication and mention
of the grant of the patent:
**24.03.2004 Bulletin 2004/13**

(51) Int Cl.[7]: **A23L 1/222**, A61K 31/35,
A21D 13/00, C07D 311/30,
C07H 17/07

(21) Application number: **99944890.5**

(22) Date of filing: **15.09.1999**

(86) International application number:
**PCT/KR1999/000548**

(87) International publication number:
**WO 2000/015047 (23.03.2000 Gazette 2000/12)**

(54) **USE OF NEOHESPERIDIN DIHYDROCHALCONE FOR THE MANUFACTURE OF A MEDICAMENT FOR PREVENTING OR TREATING ELEVATED BLOOD LIPID LEVEL-RELATED DISEASES**

VERWENDUNG VON NEOHESPERIDIN DC ZUR HERSTELLUNG EINES MEDIKAMENTS ZUR VORBEUGUNG BZW. BEHANDLUNG VON KRANKHEITEN AUFGRUND ERHOEHTER BLUTFETTWERTE

UTILISATION DE NEOHESPERIDINE DIHYDROCHALCONE DANS LA PREPARATION D'UN MEDICAMENT POUR LA PREVENTION OU LE TRAITEMENT DE MALADIES LIEES A DES NIVEAUX ELEVES DE LIPIDE DANS LE SANG

(84) Designated Contracting States:
**DE FR GB IT**

(30) Priority: **15.09.1998 KR 9837963**

(43) Date of publication of application:
**11.07.2001 Bulletin 2001/28**

(73) Proprietor: **Korea Research Institute of Bioscience and Biotechnology Daejeon 305-333 (KR)**

(72) Inventors:
• **BOK, Song, Hae**
  **Seo-gu Daejeon 302-222 (KR)**
• **JEONG, Tae, Sook**
  **Yuseong-gu Daejeon 305-333 (KR)**
• **BAE, Ki, Hwan**
  **Seo-gu Daejeon 302-200 (KR)**
• **PARK, Yong, Bok**
  **Suseong-gu Daegu 706-014 (KR)**
• **CHOI, Myung, Sook**
  **Suseong-gu Daegu 706-014 (KR)**
• **MOON, Surk, Sik**
  **Haeundae-gu, Pusan 612-021, (KR)**
• **KWON, Yong, Kook**
  **Yuseong-gu Daejeon 305-333 (KR)**
• **LEE, Eun, Sook**
  **Jung-gu Daejeon 301-013 (KR)**
• **HYUN, Byung, Hwa**
  **Yuseong-gu Daejeon 305-333 (KR)**
• **CHOI, Yang, Kyu**
  **Yuseong-gu,Daejeon 305-333 (KR)**
• **LEE, Chul, Ho**
  **Seo-gu Daejon 302-171 (KR)**
• **LEE, Sae, Bom**
  **Yuseong-gu Daejeon 305-333 (KR)**
• **PARK, Young, Bae**
  **Kangnam-gu Seoul 135-110 (KR)**
• **KIM, Hyo, Soo**
  **Kangnam-gu Seoul 135-110 (KR)**

(74) Representative: **Lorenz, Werner, Dr.-Ing.
Lorenz & Kollegen
Patent- und Rechtsanwaltskanzlei
Alte Ulmer Strasse 2-4
89522 Heidenheim (DE)**

(56) References cited:
**US-A- 3 689 663**

• **EC SWEETENERS DIRECTIVE (94/35/EC), [Online] 1994, pages 1 - 4 Retrieved from the Internet: <URL:http://www.daniscosugar.com/dssw/eng/a bout_suger/sweetlex/lagar.htm>**
• **PATENT ABSTRACTS OF JAPAN vol. 1996, no. 05 31 May 1996 & JP 08 027 006 A (TANABE SEIYAKU CO LTD)**

• **PATENT ABSTRACTS OF JAPAN vol. 1997, no. 10 31 October 1997 & JP 09 143 070 A (NIPPON FLOUR MILLS CO LTD)**

• **EISENBRAND G.; SCHREITER P.: 'Römpp Lexikon Lebensmittelchemie', 1995, GEORG THIEME VERLAG, STUTTGART**

**Description**

The present invention relates to the use of neohesperidin dihydrochalcone for the manufacture of a medicament for treating or preventing an elevated blood lipid level-related disease in a mammal.

BACKGROUND OF THE INVENTION

It has been reported that blood lipids, especially cholesterols and triglycerides, are closely related to various kind of diseases such as coronary cardio-circulatory diseases, e.g., arteriosclerosis and hypercholesterolemia, and fatty liver. Cholesterol, a fatty steroid alcohol, is a blood lipid produced from saturated fat in the liver. Triglycerides are another type of blood lipids which are known to increase the risk of various diseases. It has also been reported that an elevated blood or plasma cholesterol level causes the deposition of fat, macrophages and foam cells on the wall of blood vessels, such deposit leading to plaque formation and then to arterioscleosis (see Ross, R., Nature, 362, 801-809(1993)). One of the methods for decreasing the plasma cholesterol level is alimentotherapy to reduce the ingestion of cholesterol and lipids. Another method is to inhibit the absorption of cholesterol by inhibiting enzymes involved therein.

EC sweeteners directive 94/35/EC (June 1994) describes the use of neohesperidin as a sweetener in the food sector. Furthermore, from RÖMPP Lexikon Lebensmittelchemie, Stuttgart: Georg Thieme Verlag, 1995, page 218, the high intensity of neohesperidin dihydrochalkone as a sweetener is known.

Acyl CoA-cholesterol-o-acyltransferase (ACAT) promotes the esterification of cholesterol in blood. Foam cells are formed by the action of ACAT and contain a large amount of cholesterol ester carried by low density lipoproteins. The formation of foam cells on the wall of artery increases with the ACAT activity, and, accordingly, an inhibitor of ACAT may also be an agent for preventing arteriosclerosis. Further, it has been reported that the blood level of LDL-cholesterol can be reduced by inhibiting the ACAT activity(see Witiak, D. T. and D. R. Feller(eds.), Anti-Lipidemic Drugs: Medicinal, Chemical and Biochemical Aspects, Elsevier, pp159-195(1991)).

Further, it has been reported that hypercholesterolemia can be treated effectively by reducing the rate of cholesterol biosynthesis through the inhibition of cholesterol ester transfer protein(CETP) which mediates the cholesterol transfers between the lipoproteins, or 3-hydroxy-3-methylglutaryl coenzyme A(HMG-CoA) reductase which mediates the synthesis of mevalonic acid, an intermediate in the biosynthesis of sterols or isoprenoids(see Cardiovascular Pharmacology, William W. Parmley and Kanu Chatterjee Ed., Wolfe Publishing, pages 8.6-8.7, 1994).

Therefore, numerous efforts have been made to develop medicines to inhibit HMG-CoA reductase; and, as a result, several compounds derived from Penicillium sp. and Aspergillus sp. have been commercialized. Specifically, Lovastatin® and Simvastatin® developed by Merck Co., U.S.A., and Pravastatin® developed by Sankyo Co., Japan, have been commercialized(see C.D.R. Dunn, Stroke: Trends, Treatment and Markets, SCRIPT Report, PJB Publications Ltd., 1995).

However, these medicines are very expensive and a long-term administration thereof is known to induce an adverse side effect to the central nervous system. Further, although Lovastatin® and Simvastatin® may reduce the plasma LDL cholesterol level by enhancing the activity of LDL receptor in the liver, they cause side effects such as increase in creatine kinase in the liver and rhabdomyolysis(see Farmer, J.A., et al., Baillers-clin. Endocrinol. Metal., 9, 825-847(1995)). Accordingly, there has continued to exist a need to develop an inexpensive and non-toxic inhibitor of HMG-CoA reductase.

Another example of the elevated blood-lipid level-related disease is fatty liver. In particular, the excessive intake of fat-containing foods and alcohol causes-fatty liver wherein a large amount of lipids is deposited in the liver tissue and the levels of serum GOT(glutamate-oxaloacetate transaminase), GPT(glutamate-pyruvate transaminase) and γ-GTP(γ-glutamyl transpeptidase) are elevated(see T. Banciu et al., Med. Interne., 20, 69-71(1982); and A. Par et al., Acta. Med. Acad. Sci. Hung., 33, 309-319(1976)).

Fat accumulates in the liver mainly in the form of triglycerides and fatty acids, and also to a minor extent, in the form of cholesterol. Further, it has been reported that one of the major signs of fatty liver is high blood cholesterol and/or triglyceride contents. Therefore, fatty liver is closely related to the level of cholesterol and/or triglycerides in the blood.

Bioflavonoids are polyphenolic antioxidants which exist widely in the natural world, especially in vegetables, fruits, wine and the like. It has been reported that the bioflavonoids exhibit various useful pharmacological activities such as anti-inflammatory, capillary reinforcing, anti-oxidative, anti-cancer, anti-viral and anti-platelet aggregation activities (see O. Benavente-Garcia et al., Uses and properties of citrus flavonoids, J. Agr. Food Chem., 45, 4506-4515, 1997).

Representative bioflavonoids, which can be found in citruses, are listed in Table I.

Table I

| Citrus fruit | Bioflavonoids |
| --- | --- |
| Grapefruit | apigenin, dihydrokaempferol, eriodictyol, hesperetin, hesperidin, isorhamnetin, isosakuranetin, kaempferol, naringenin, naringin, neohesperidin, poncirin, quercetin, rutin |
| Lemon | apigenin, apigenin 7-rutinoside, chrysoeriol, diosmin, eriocitrin, hesperidin, isorhamnetin, limocitrin, limocitrol, luteolin 7-rutinoside, naringin, neohesperidin, poncirin, quercetin . |
| Orange | auranetin, hesperidin, isosakuranetin 7-rutinoside, naringin, neohesperidin, nobiletin, rutin, sinensetin, tangeretin, vitexin |
| Tangerine | hesperidin, nobiletin, tangeretin |

Neohesperidin dihydrochalcone, a bioflavonoid which can be easily extracted from grapefruit or synthesized from naringin, is known to have a 1,000 to 1,500 fold higher sweetness than sucrose.

The present inventors have endeavored to develop a novel pharmacological use of bioflavonoids which are abundantly present in herbs, foodstuffs, vegetables and fruits. As a result, it has been discovered that neohesperidin dihydrochalcone is effective in treating or preventing elevated blood lipid level-related diseases. Specifically, it can greatly reduce plasma cholesterol level; prevent the activities of HMG-CoA reductase and ACAT; inhibit the accumulation of macrophage-lipid complex on the endothelial wall of an artery and prevent hepatic dysfunctions in a mammal.

SUMMARY OF THE INVENTION

Accordingly, it is an object of the present invention to provide the use of neohesperidin dihydrochalcone for the manufacture of a medicament according to claim 1.

BRIEF DESCRIPTION OF THE DRAWINGS

The above and other objects and features of the present invention will become apparent from the following description of the invention, when taken in conjunction with the accompanying drawings, in which:

Figs. 1A, 1B and 1C show the arterial endothelium of the rabbits administered with 1% cholesterol; 1% cholesterol plus 1 mg/kg Lovastatin®; and 1% cholesterol plus 0.05% neohesperidin dihydrochalcone, respectively; and
Figs. 2A, 2B and 2C present the microscopic features of the livers of the rabbits administered with 1% cholesterol; 1% cholesterol plus 1 mg/kg Lovastatin®; and 1% cholesterol plus 0.05% neohesperidin dihydrochalcone, respectively.

DETAILED DESCRIPTION OF THE INVENTION

Throughout the specification, the term "blood lipid"
designates a lipid present in the blood. The blood lipid is represented by cholesterols and triglycerides carried in the blood.

The term "high or elevated level" of a blood lipid means higher than normal level, the normal level varying with specific conditions of a patient, such as age, gender and body weight. A high level of blood lipid is ordinarily considered to be harmful to health.

The term "elevated blood lipid level-related disease" means a disease which is caused by a high or elevated level of blood lipid, and/or a disease whose symptoms include a high or elevated level of blood lipid. Examples of such a disease include hyperlipidemia, arteriosclerosis, angina pectoris, stroke and hepatic diseases such as fatty liver.

Neohesperidin dihydrochalcone ($C_{28}H_{36}O_{15}$, M.W. 612.60, see Merck Index 11th ed. (1989) ) can be easily extracted from the peel of grapefruit, or synthesized from naringin in accordance with a conventional process.

Neohesperidin dihydrochalcone exerts inhibitory as well as therapeutic effects on elevated blood lipid level-related diseases, e.g., hyperlipidemia, arteriosclerosis, angina pectoris, stroke and hepatic diseases. Further, in spite of its potent efficacy, neohesperidindihydrochalcone exhibits no toxicity when it is orally administered to a mouse at a dose of 1,000 mg/kg. Moreover, it does not adversely affect on the liver function.

A pharmaceutical formulation may be prepared in accordance with any of the conventional procedures. In preparing the formulation, the active ingredient is preferably admixed or diluted with a carrier, or enclosed within a carrier which may be in the form of a capsule, sachet or other container. When the carrier serves as a diluent, it may be a solid, semi-solid or liquid material acting as a vehicle, excipient or medium for the active ingredient. Thus, the formulations may be in the form of a tablet, pill, powder, sachet, elixir, suspension, emulsion, solution, syrup, aerosol, soft and hard gelatin capsule, sterile injectable solution, sterile packaged powder and the like.

Examples of suitable carriers, excipients, and diluents are lactose, dextrose, sucrose, sorbitol, mannitol, starches, gum acacia, alginates, gelatin, calcium phosphate, calcium silicate, cellulose, methyl cellulose, microcrystalline cellulose, polyvinylpyrrolidone, water, methylhydroxy-benzoates, propylhydroxybenzoates, talc, magnesium stearate and mineral oil. The formulations may additionally include fillers, anti-agglutinating agents, lubricating agents, wetting agents, flavoring agents, emulsifiers, preservatives and the like. The compositions of the invention may be formulated so as to provide quick, sustained or delayed release of the active ingredient after their administration to a mammal by employing any of the procedures well known in the art.

The pharmaceutical composition of the present invention can be administered via various routes including oral, transdermal, subcutaneous, intravenous and intramuscular introduction. In case of human, a typical daily dose of neohesperidin dihydrochalcone may range from about 0.1 to 500 mg/kg body weight, preferably 1 to 100 mg/kg body weight, and can be administered in a single dose or in divided doses.

However, it should be understood that the amount of the active ingredient actually administered ought to be determined in light of various relevant factors including the condition to be treated, the chosen route of administration, the age, sex and body weight of the individual patient, and the severity of the patient's symptom.

Moreover, neohesperidin dihydrochalcone can be advantageously incorporated in foods or beverages for the purpose of treating or preventing elevated blood lipid level-related diseases, e.g., hyperlipidemia, arteriosclerosis, angina pectoris, stroke and hepatic diseases. The foods or beverages may include meats; juices such as a vegetable juice(e.g., carrot juice and tomato juice) and a fruit juice(e.g., orange juice, grape juice, pineapple juice, apple juice and banana juice); chocolates; snacks; confectionery; pizza; food products made from cereal flour such as breads, cakes, crackers, cookies, biscuits, noodles and the likes; gums; dairy products such as milk, cheese, yogurt and ice creams; soups; broths; pastes, ketchups and sauces; teas; alcoholic beverages; carbonated beverages; vitamin complexes; and various health foods.

The content of the neohesperidin dihydrochalcone in a food or beverage may range from 0.01 to 20 wt%, preferably, from 0.1 to 5 wt%.

As described above, neohesperidin dihydrochalcone can be used as an effective, non-toxic pharmaceutical agent for treating or preventing elevated blood lipid level-related diseases, e.g., hyperlipidemia, arteriosclerosis, angina pectoris, stroke and hepatic diseases.

The following Examples are intended to further illustrate the present invention.

Further, percentages given below for solid in solid mixture, liquid in liquid, and solid in liquid are on a wt/wt, vol/vol and wt/vol basis, respectively, and all the reactions were carried out at room temperature, unless specifically indicated otherwise.

Example 1: Toxicity of Orally Administered Neohesperidin Dihydrochalcone

12 seven-week-old, specific pathogen-free ICR female mice, six female mice each weighing about 25 to 29 g and six male mice each weighing about 34 to 38 g, were kept under an environment of 22±1°C, 55±5 % relative humidity and 12L/12D photoperiod. Fodder(Cheiljedang Co., mouse and rat fodder) and water were sterilized and fed to the mice.

Neohesperidin dihydrochalcone purchased from Aldrich-Sigma Chemical Co. (St. Louis, MO, U.S.A) was dissolved in 0.5 % Tween 80 to a concentration of 100 mg/ml, and the solution was orally administered to the mice in an amount of 0.2 ml per 20 g of mouse body weight. The solution was administered once and the mice were observed for 10 days for signs of adverse effects or death according to the following schedule: 1, 4, 8, and 12 hours after the administration and, every 12 hours thereafter, the weight changes of the mice were recorded to examine the effect of neohesperidin dihydrochalcone. Further, on the 10th day, the mice were sacrificed and the internal organs were visually examined.

All the mice were alive at day 10 and neohesperidin dihydrochalcone showed no toxicity at a dose of 1,000 mg/kg. The autopsy revealed that the mice did not develop any pathological abnormality, and no weight loss was observed during the 10 day test period. Accordingly, it was concluded that neohesperidin dihydrochalcone is not toxic when orally administered to an animal.

Example 2: Effect of Neohesperidin Dihydrochalcone on Plasma Cholesterol, HDL-Cholesterol and Neutral Lipid Levels

(Step 1) Administration of neohesperidin dihydrochalcone to rats

20 three-week-old white Sprague-Dawley rats(Taihan laboratory animal center, Korea) , each weighing about 9.0 to 110 g, were evenly divided into two dietary groups by a randomized block design. The rats of the two groups were fed with two different high-cholesterol diets, i.e., AIN-76 laboratory animal diet(ICN Biochemicals, Cleveland, OH, U.S.A.) containing 1 % cholesterol(Control group) and 1 % cholesterol plus 0.05 % neohesperidin dihydrachalcone (Neohesperidin dihydrochalcone group), respectively. The compositions of the diets fed to the two groups are shown in Table II.

Table II

| Dietary group Component | Control (n=10) | Neohesperidin dihydrochalcone (n=10) |
|---|---|---|
| Casein | 20 | 20 |
| D,L-methionine | 0.3 | 0.3 |
| Corn starch | 15 | 15 |
| Sucrose | 49 | 48.95 |
| Cellulose powder*1 | 5 | 5 |
| Mineral mixture*1 | 3.5 | 3.5 |
| Vitamin mixture*1 | 1 | 1 |
| Choline citrate | 0.2 | 0.2 |
| Corn oil | 5 | 5 |
| Cholesterol | 1 | 1 |
| Neohesperidin dihydrochalcone*2 | - | 0.05 |
| Total | 100 | 100 |

*1 Purchased from TEKLAD premier Co. (Madison, WI, U.S.A.)
*2 Purchased from Sigma Chemical Co. (St. Louis, MO, U.S.A.)

The rats were allowed to feed freely on the specified diet together with water for six weeks, the ingestion amount was recorded daily and the rats were weighed every 7 days, and then the record was analyzed. All rats showed a normal growth rate and there was observed no significant difference among the two groups in terms of the feed ingestion amount and the weight gain.

(Step 2) Determination of total cholesterol, HDL-cholesterol and neutral lipid content in blood

The effects of administering neohesperidin dihydrochalcone to rats on the plasma cholesterol and neutral lipid contents were determined as follows.
The rats of the two dietary groups obtained in Step 1 were sacrificed and blood samples were taken therefrom. The blood was allowed to stand for 2 hours and centrifuged at-3,000 rpm for 15 minutes and the supernatant was separated and stored in a deep freezer before use. The chemical analysis of blood was carried out by employing a blood chemical analyzer(CIBA Corning 550 Express, USA) to determine the changes in total cholesterol, HDL-cholesterol and triglyceride levels. The result is shown in Table III.

Table III

| Group Lipid Conc. | Control | Neohesperidin dihydrochalcone |
|---|---|---|
| Total-C (mg/dl) | 135±28 | 115±10 |
| HDL-C (mg/dl) | 18±4 | 19±3 |
| TG (mg/dl) | 57±13 | 52±8 |

Table III   (continued)

| Group Lipid Conc. | Control | Neohesperidin dihydrochalcone |
|---|---|---|
| $\dfrac{\text{HDL-C}}{\text{Total-C (\%)}}$ | 13 | 16 |
| * Total-C: Total-cholesterol | | |
| * HDL-C: HDL-cholesterol | | |
| * TG: Triglyceride | | |

As can be seen from Table III, the total plasma cholesterol level is reduced by 15 % in the Neohesperidin dihydrochalcone group, as compared with that of the Control group. Further, the neutral lipid content is reduced by 9 % in the Neohesperidin dihydrochalcone group, as compared with that of the Control group.

Example 3: Activity of Neohesperidin dihydrochalcone in ACAT Inhibition

(Step 1) Preparation of microsomes

To determine the effect of feeding neohesperidin dihydrochalcone to rats on the activity of ACAT, microsomes were separated from liver tissues to be used as an enzyme source.
1 g each of the livers taken from each group of rats of Example 2 was homogenized in 5 ml of homogenization medium(0.1 M $KH_2PO_4$, pH 7.4, 0.1 mM EDTA and 10 mM β-mercaptoethanol). The homogenate was centrifuged at 3,000xg for 15 min. at 4°C and the supernatant thus obtained was centrifuged at 15,000xg for 15 min. at 4°C to obtain a supernatant. The supernatant was put into an ultracentrifuge tube(Beckman) and centrifuged at 100,000xg for 1 hour at 4°C to obtain microsomal pellets, which were then suspended in 3 ml of the homogenization medium and centrifuged at 100,000xg for 1 hour at 4°C. The pellets thus obtained were suspended in 1 ml of the homogenization medium. The protein concentration of the resulting suspension was determined by Lowry's method and then adjusted to 4 to 8 mg/ ml. The resulting suspension was stored in a deep freezer (Biofreezer, Forma Scientific Inc.).

(Step 2) ACAT assay

6.67 µl of 1 mg/ml cholesterol solution in acetone was mixed with 6 µl of 10 % Triton WR-1339(Sigma Co.) in acetone and, then, acetone was removed from the mixture by evaporation under a nitrogen flow. Distilled water was added to the resulting mixture to adjust the concentration of cholesterol to 30 mg/ml.
Added to 10 µl of the resulting aqueous cholesterol solution were 10 µl of 1 M $KH_2PO_4$(pH 7.4), 5 µl of 0.6 mM bovine serum albumin(BSA), 10 µl of microsome solution obtained in (Step 1) and 55 µl of distilled water(total 90 µl). The mixture was pre-incubated in a water bath at 37°C for 30 min.
10 µl of (1-$^{14}$C) oleyl-CoA solution(0.05 µCi, final concentration: 10 µM) was added to the pre-incubated mixture and the resulting mixture was incubated in a water bath at 37°C for 30 min. Added to the mixture were 500 µl of isopropanol:heptane mixture (4:1 (v/v)), 300 µl of heptane and 200 µl of 0.1 M $KH_2PO_4$(pH 7.4), and the mixture was mixed vigorously using a vortex mixer and then allowed to stand at room temperature for 2 min.
200 µl of the resulting supernatant was put in a scintillation bottle and 4 ml of scintillation fluid(Lumac) was added thereto. The mixture was assayed for radioactivity with 1450 Microbeta liquid scintillation counter(Wallacoy, Finland). ACAT activity was calculated as picomoles of cholesteryl oleate synthesized per min. per mg protein(pmoles/ min/mg protein). The result is shown in Table IV.

Table IV

| Group | %Inhibition on ACAT activity |
|---|---|
| Control | 0 |
| Neohesperidin dihydrochalcone | 20 |

As can be seen from Table IV, ACAT activity observed in the Neohesperidin dihydrochalcone group is lower than that of the Control group by 20 %.

Example 4: Activity of Neohesperidin dihydrochalcone in HMG-CoA Reductase Inhibition

In order to determine the activity of HMG-CoA reductase, Hulcher's method was employed after some modification (see J. Lipid Res., 14, 625-641(1973)). In this method, the concentration of the coenzyme-A(CoA-SH), which is produced when HMG-CoA is reduced to a mevalonate salt by the action of HMG-CoA reductase, is determined by spectroscopy and the activity of HMG-GoA reductase is calculated therefrom.

(Step 1) Preparation of microsomes

3 g of liver tissue taken from each group of rats of Example 2 was washed succesively with 100 ml of a cold-saline(0.15M NaCl) and 100ml of a cold buffer solution A(0.1M triethanolamine, HCl/0.2M EDTA/2mM dithiothreitol (DTT)). The cold buffer solution A was added to the liver tissue in an amount of 2 ml per 1 g of the liver tissue and the mixture was homogenized with a homogenizer. The homogenate was centrifuged at 15,000xg for 15 minutes, and then, the supernatant was ultracentrifuged at 100,000xg for 60 minutes to obtain microsomal precipitates. The precipitates thus obtained was washed with a cold buffer solution A and kept in a 1.5ml tube at -70°C.

(Step 2) HMG-CoA reductase activity assay

The reaction substrates used in HMG-CoA reductase activity assay were as follows: i) buffer solution B: 0.1M triethanolamine, HCl/0.02M EDTA(pH7.4), ii) HMG-CoA solution: 150 µmoles/culture medium, and iii) NADPH solution : 2 µmoles/culture medium.

The suspension(miorosome) was mixed with the reaction substrate and the mixture was placed in a centrifugation tube and reacted at 37°C for 30 minutes. The reaction mixture was treated with 20µl of 0.01M sodium arsenous and allowed to stand for 1 minute, and then it was reacted with 100µl of citrate buffer solution(2M citrate/3% sodium tungstate, pH 3.5) at 37°C for 10 minutes followed by centrifugation at 25,000xg for 15 minutes to remove protein. 1ml of the supernatant thus obtained was transferred into a tube with a cap and added thereto were 0.1ml of 2M tris-HCl solution(pH 10.6) and 0.1ml of 2M tris-HCl solution(pH 8.0) to adjust the pH of the reactant to 8.0.

Then, the reactant was mixed with 20µl of DTNB buffer solution(3mM DTNH/0.1M triethanolamine/0.2M EDTA, pH 7.4) and the absorbance of the mixture was determined at 412nm to calculate the amount of CoA-SH(activity of HMG-CoA reductase).

The extent of inhibition of HMG-CoA reductase activity by neohesperidin dihydrochalcone was calculated based on the above result. The result is shown in Table V.

Table V

| Group | Inhibition of HMG-CoA reductase activity (%) |
|---|---|
| Control | 0 |
| Neohesperidin dihydrochalcone | 30 |

As can be seen in Table V, the HMG-CoA reductase activity observed in the Neohesperidin dihydrochalcone group is lower than that of the Control group by 30 %.

Example 5: Effect of Administration of Neohesperidin Dihydrochalcone to a Human on Plasma Lipid Metabolism

Two men in their mid-fifties were administered with a daily oral dose of 10mg/kg of neohesperidin dihydrochalcone in the form of a capsule for 60 days. The plasma cholesterol and neutral lipid(triglyceride) contents were determined before and after the administration.

The plasma cholesterol and neutral lipid contents were reduced by the neohesperidin dihydrochalcone administration by 20 % and 15 %, respectively.

Example 6: Inhibition of Arteriosclerosis

(Step 1) Administration of neohesperidin dihydrochalcone to rabbits

30 three-month-old New Zealand White rabbits(Yeonam Horticulture and Animal Husbandry College, Korea), each weighing about 2.5 to 2.6 kg, were raised under a condition of temperature 20±2°C, relative humidity 55±5 %, and photoperiod 12L/12D. The rabbits were divided into 3 groups and 3 groups of rabbits were fed with 3 different diets, i.e., RC4 diet(Oriental Yeast Co., Japan) containing 1 % cholesterol(Control group); 1 % cholesterol plus 1 mg/kg Lovastatin®(Merck, U.S.A.)(Lovastatin group); and 1 % cholesterol plus 0.05 % neohesperidin dihydrochalcone (Neohesperidin dihydrochalcone group), respectively. RC4 diet comprises 7.6 % moisture, 22.8 % crude protein, 2.8 % crude fat, 8.8 % crude ash, 14.4 % crude cellulose and 43.6 % soluble nitrogen-free substances. Neohesperidin dihydrochalcone was purchased from Sigma Chemical Co.(St. Louis, MO).

The rabbits were fed for 6 weeks while being allowed free access to the diets and water.

(Step 2) Chemical Analysis of Blood

After six weeks, the rabbits were anesthetized with an intramuscular injection of ketamine (50 mg/kg) in the femoral region and sacrificed. A blood sample was taken from the heart of each rabbit, allowed to stand for 2 hours and centrifuged at 3,000 rpm for 15 minutes and the supernatant serum was separated and stored in a freezer before use.

The chemical analysis of blood was carried out by employing a blood chemical analyzer(CIBA Corning 550 Express, USA) to determine the changes in GOT, GPT, $\gamma$-GTP and total cholesterol levels. The results are shown in Table VI.

(Step 3) Analysis for fatty streak in the main artery

The chest of each of the rabbits sacrificed in Step 2 was incised. The downward portion of the main artery from the site 1 cm above the aortic valve was cut out in a length of about 5 cm and the fat surrounding the main artery was removed. The main artery was incised in the middle along the longitudinal axis and pinned to a dish. The moist artery was photographed and, then, the staining of fatty streaks was carried out in accordance with the method of Esper, E., et al. (J. Lab. Clin. Med., 121, 103-110(1993)) as follows.

A part of the incised main artery was washed three times with anhydrous propylene glycol for 2 min and stained for 30 min. with a saturated solution of Oil Red O(ORO, Sigma Co.) dissolved in propylene glycol. Thereafter, the artery was washed twice with 85 % propylene glycol for 3 min. to remove remaining staining solution and, then washed with physiological saline. The artery was photographed and the photograph was traced. The area of stained region (fatty streak region) was determined with an image analyzer(LEICA, Q-600, Germany) and its proportion(%) to the total arterial area was calculated. The result is shown in Table VI.

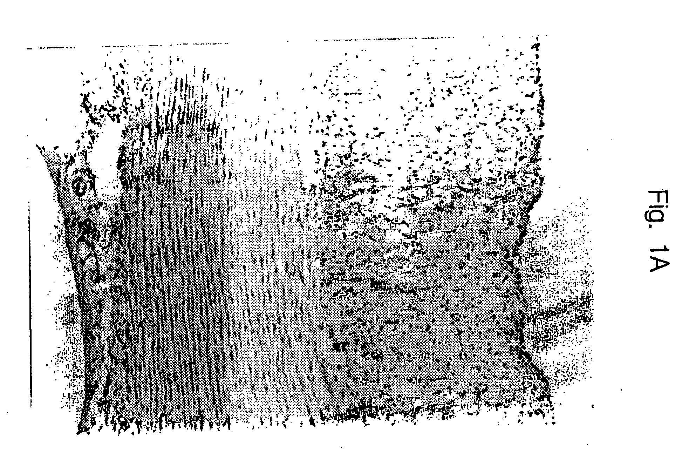

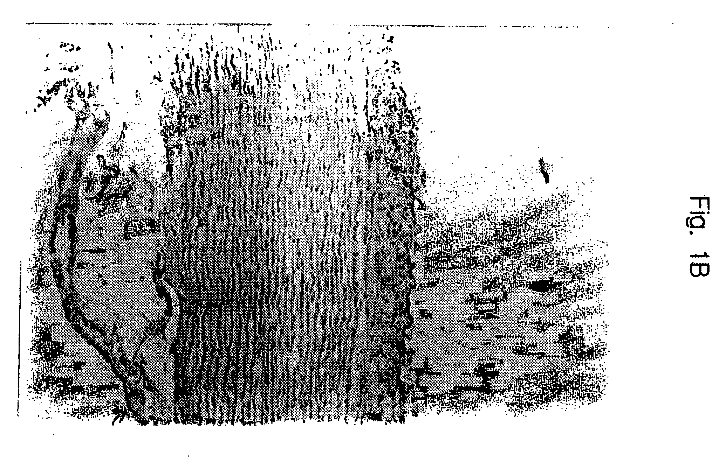

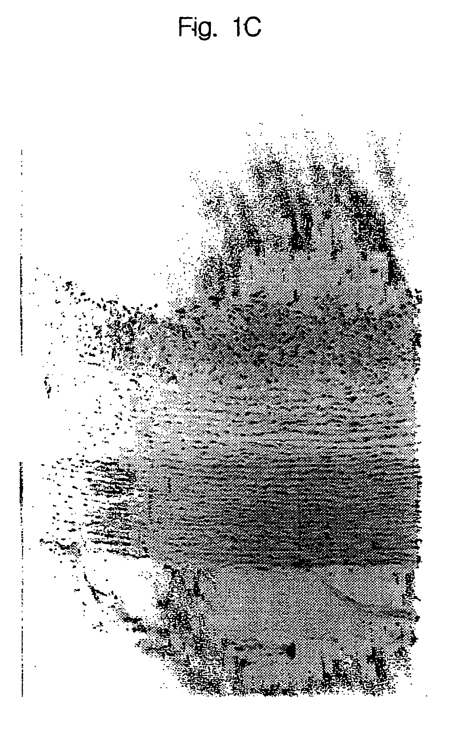

Figs. 1A, 1B and 1C show the arteries of the rabbits administered with 1 % cholesterol(Control group); 1 % cholesterol plus 1 mg/kg Lovastatin®((Lovastatin group); and 1 % cholesterol plus 0.05 % neohesperidin dihydrochalcone(Neohesperidin dihydrochalcone group), respectively. As shown in Figs. 1A, 1B and 1C, a thick layer of macrophage-lipid complex was observed on the arterial endothelium of the rabbit administered with 1 % cholesterol, while no or very thin layers of macrophage-lipid complex were observed on the arterial endothelia of the rabbits administered with 1 % cholesterol plus 1 mg/kg Lovastatin® and 1 % cholesterol plus 0.05 % neohesperidin dihydrochalcone, respectively.

Accordingly, it is concluded that the neohesperidin dihydrochalcone composition of the present invention strongly inhibits the deposition of macrophages on the arterial endothelium.

(Step 4) Histologic observation of the organs

Portions of the main artery, heart, lung, liver, kidney and muscle were taken from each of the rabbits sacrificed in step 2 and visually examined to confirm that no pathogenic abnormality was found. One half of each portion of the organs was deep freezed and the other half was fixed in 10 % neutral buffered formalin for more than 24 hours. The fixed organ piece was washed sufficiently with tap water, dehydrated stepwise with 70 %, 80 %, 90 % and 100 % ethanol and, then, embedded in a paraffin by employing SHANDON®, Histocentre 2, USA. The embedded organ piece was sectioned in 4 $\mu$m thickness with a microtome(LSICA, RM2045, Germany) and stained with hematoxylin and eosin. The stained organ specimen was made transparent with xylene, mounted with permount, and then observed under a microscope to look for the presence of lesions. No lesion was observed in any of the organ specimen.

Example 7: Prevention of Hepatic Diseases

In order to evaluate the effects of feeding a high cholesterol diet with neohesperidin dihydrochalcone on liver tissues, the liver specimens taken from the sacrificed rabbit in Step 2 of Example 6 were treated in accordance with the procedure disclosed in Fogt F. and Nanji A., Toxicology and Applied Pharmacology, 136, 87-93, 1996; and Keegan A., et al., Journal of Hepatology 23: 591-600, 1995, and observed under a microscope to be classified into four grades, i.e., 1+(0-25%), 2+(26-50%), 3+(51-75), 4+(76-100%) based on the proportion of abnormal fat-containing cells around the central vein in the liver acinus. The result is shown in Table VI.

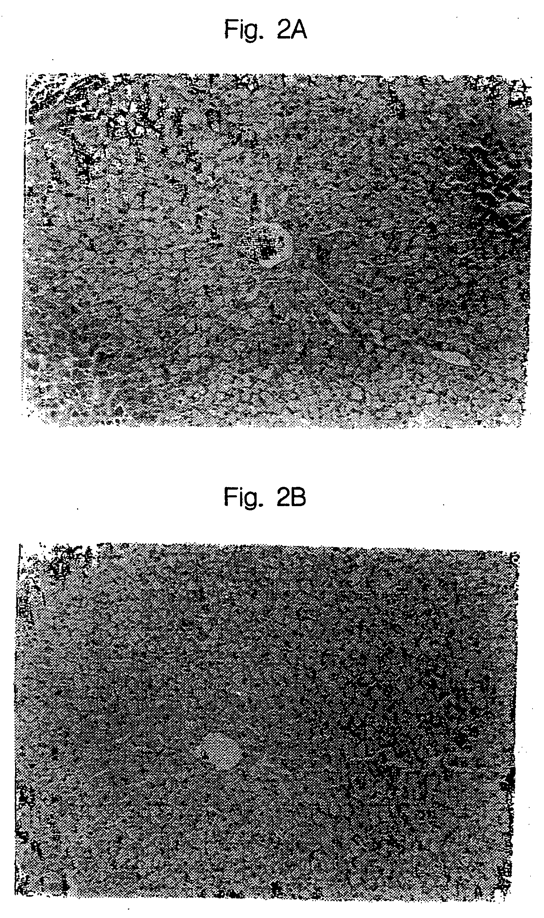

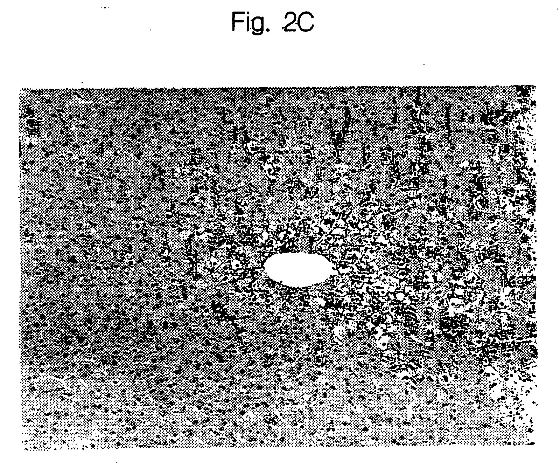

Figs. 2A, 2B and 2C present the microscopic features of the livers of the rabbits administered with 1% cholesterol; 1% cholesterol plus 1 mg/kg Lovastatin®; 1% and cholesterol plus 0.05% neohesperidin dihydrochalcone, respectively. In Figs. 2A and 2B, many cells containing excessive fat were observed around the central vein. In contrast, almost all liver cells are of a normal shape in Fig. 2C, which suggested that neohesperidin dihydrochalcone can significantly inhibit the formation of fatty liver.

As can be seen from the above, the administration of neohesperidin dihydrochalcone can improve lipid metabolism in rabbit and liver function and inhibit the plaque formation in the endothelium of the main artery and formation of fatty liver as shown in Table VI. The results were tested by student t-test by using Microsoft excel(version 7.0) program.

Table VI

| Group | Total-C (mg/dl) | GOT (IU/1) | GPT (IU/l) | $\gamma$-GTP (IU/l) | A(%) | B |
|---|---|---|---|---|---|---|
| Control | 1143 (±260) | 77 (±8) | 65 (±9) | 4.2 (±1) | 35 (±14) | 3.2g (±0.3) |
| Lovastatin | 1210 (±263) | 125 (±19) | 73 (±9) | 12.4 (±0.8) | 5 (±4) | 3.4 (±0.5) |
| Neohesperidin dihydrochalco ne | 1293 (±89) | 53 (±23) | 42 (±20) | 7.8 (±5) | 12 (±7) | 2.7 (±0.3) |
| * Total-C: Total-cholesterol A: Proportion(%) of fatty streak region to the total arterial area B: Proportion of abnormal fat-containing cells | | | | | | |

As can be seen from Table VI, administration of neohesperidin dihydrochalcone lowers serum GOT and GPT levels by 31 % and 35 %, respectively, as compared to the Control group. Especially, neohesperidin dihydrochalcone is more effective in reducing serum GOT and GPT levels than Lovastatin®.

Formulation 1: Preparation of Pharmaceutical Formulation

Hard gelatin capsules were prepared using the following ingredients:

| | Quantity(mg/capsule) |
|---|---|
| Active ingredient (neohesperidin dihydrochalcone) Vitamin C Lactose(carrier) | 200 50 150 |
| Total | 400mg |

Formulation 2: Foods Containing Neohesperidin Dihydrochalcone

Foods containing neohesperidin dihydrochalcone were prepared as follows.

(1) Preparation of tomato ketchup and sauce
Neohesperidin dihydrochalcone was added to a tomato ketchup or sauce in an amount ranging from 0.01 to 10 wt% to obtain a health-improving tomato ketchup or sauce.

(2) Preparation of foods containing wheat flour
Neohesperidin dihydrochalcone was added to wheat flour in an amount ranging from 0.01 to 10 wt% and breads, cakes, cookies, crackers and noodles were prepared by using the mixture to obtain health-improving foods.

(3) Preparation of soups and gravies
Neohesperidin dihydrochalcone was added to soups and gravies in an amount ranging from 0.01 to 10 wt% to obtain health-improving soups and gravies.

(4) Preparation of ground beef
Neohesperidin dihydrochalcone was added to ground beef in an amount ranging from 0.01 to 10 wt% to obtain health-improving ground beef.

(5) Preparation of dairy products

Neohesperidin dihydrochalcone was added to milk in an amount ranging from 0.01 to 10 wt% to obtain health-improving milk, and various dairy products such as butter and ice cream were prepared therefrom.

In case of a cheese preparation, neohesperidin dihydrochalcone was added to coagulated milk protein; and, in case of a yogurt preparation, neohesperidin dihydrochalcone was added to coagulated milk protein obtained after the fermentation.

Formulation 3: Beverages Containing Neohesperidin Dihydrochalcone

(1) Preparation of vegetable juice

100 to 5,000 mg of neohesperidin dihydrochalcone was added to 1000 ml of a vegetable juice to obtain a health improving vegetable juice.

(2) Preparation of fruit juice

100 to 5,000 mg of neohesperidin dihydrochalcone was added to 1000 ml of a fruit juice to obtain a health-improving fruit juice.

Formulation 4: Health Foods Containing Neohesperidin Dihydrochalcone

A health food was prepared by mixing the following ingredients and tabletting the mixture.

|  | Quantity(wt/wt%) |
| --- | --- |
| Ginseng powder or extract | 50 |
| Neohesperidin dihydrochalcone | 30 |
| Sweetener and flavor | 20 |
| Total | 100% |

**Claims**

1.  Use of neohesperidin dihydrochalcone for the manufacture of a medicament for treating or preventing an elevated blood lipid level-related disease in a mammal.

2.  The use of claim 1, wherein the disease is hyperlipidemia, arteriosclerosis, angina pectoris, stroke or fatty liver.

3.  The use of claim 1, wherein the mammal is human.

4.  The use of claim 1, wherein an effective amount of neohesperidin dihydrochalcone ranges from 0.1 to 500 mg/kg of body weight/day.

**Patentansprüche**

1.  Verwendung von Neohesperidin-Dihydrochalkon zur Herstellung eines Arzneimittels zur Behandlung oder Verhinderung einer mit einem erhöhten Blutfettwert zusammenhängenden Krankheit bei einem Säugetier.

2.  Verwendung nach Anspruch 1, wobei die Krankheit Lipidspiegelerhöhung, Arteriosklerose, Angina Pectoris, Schlaganfall oder Leberverfettung ist.

3.  Verwendung nach Anspruch 1, wobei das Säugetier ein Mensch ist.

4.  Verwendung nach Anspruch 1, wobei eine effektive Menge von Neohesperidindihydroalcon in einem Bereich von 0,1 bis 500 mg/kg des Körpergewichts/Tag beträgt.

**Revendications**

1.  Utilisation de néohespéridine dihydrochalcone pour la fabrication d'un médicament pour traiter ou prévenir une maladie liée à un taux élevé de lipides dans le sang chez un mammifère.

2.  Utilisation de la revendication 1, dans laquelle la maladie est une hyperlipidémie, une angine de poitrine, une attaque ou une stéatose hépatique.

3.  Utilisation de la revendication 1, dans laquelle le mammifère est humain.

4.  Utilisation de la revendication 1, dans laquelle la quantité effective de néohespéridine dihydrochalcone varie de 0,1 à 500 mg/kg du poids du corps par jour.

Fig. 1A

Fig. 1B

Fig. 1C

## Fig. 2A

## Fig. 2B

Fig. 2C